# EUROPEAN PATENT SPECIFICATION

(11) **EP 3 538 229 B1**
(45) Date of publication and mention of the grant of the patent: **16.06.2021**
(21) Application number: 17869513.6
(22) Date of filing: 12.11.2017
(51) Int. Cl.: A63B 23/04, A63B 24/00

(54) **PHYSIOTHERAPEUTIC STAIR&INCLINE SYSTEM**
PHYSIOTHERAPEUTISCHES STUFEN- UND NEIGUNGSSYSTEM
SYSTÈME D'ESCALIER ET DE PENTE PHYSIOTHÉRAPEUTIQUE

(30) Priority: 13.11.2016 US 201615350055
(43) Date of publication of application: 18.09.2019
(73) Proprietor: DPE Medical Ltd., 9085500 Shoeva (IL)
(72) Inventor: ORGAL, Daniel, 9085500 Shoeva (IL)
(74) Representative: Gallo, Luca
(86) International application number: PCT/IL2017/051228
(87) International publication number: WO 2018/087765

(56) References cited:
- WO-A1-2012/057595
- US-A- 3 497 215
- US-A- 5 901 813
- US-A1- 2010 099 541
- US-A1- 2015 066 171
- US-A1- 2015 141 201
- US-B1- 6 601 677
- US-B2- 7 383 600
- US-B2- 8 807 283

## Description

### FIELD AND BACKGROUND OF THE INVENTION

The present invention relates to physiotherapy devices and, in particular, it concerns a physiotherapeutic system combining stairs and an incline, and in which treatment is preferably tracked and documented by use of a computer system associated with an integrated sensor set.

Many patients require physiological rehabilitation for various reasons. These include, for example, victims of traffic accidents, patients who have suffered from a cardiac episode or underwent a cardiac medical procedure, as well as individuals that have had a cerebral episode, invasive medical procedures, or sustained injuries of violence and the like. These patients need to receive physiotherapeutic treatment in rehabilitation wards of various institutions or by professional physiotherapists, for the purpose of a gradual return to regular life.

The practicing of walking, ascending and descending stairs is a central part of the rehabilitation process. The ability to ascend and descend stairs is a vital component in the assessment process performed by the medical staff when deciding whether a patient can be discharged from the rehabilitative institution to his or her home.

PCT publication no. WO 2012/057595A1 discloses a variable stair and ramp system for rehabilitation in which a set of steps and ramp are mounted on opposite sides of a central platform of adjustable height.

One example of a suitable device for practicing ascending and descending of stairs is described in US patent number 5,901,813, and is commercially available from DPE Medical Ltd. (Israel) under the name Dynamic Stair Trainer (DST). The device consists of number of stairs whose height can be altered simultaneously according to the need and ability of the current patient.

Stair trainers used in physiotherapy for ascending and descending stairs are preferably static during use with a uniform pitch, simulating the look and feel of conventional stairs. It is also preferable that adjustment of the height occurs through a purely vertical motion without changing the depth of the tread surface of each step, and that the steps have closed riser surfaces without overlap between steps to minimize risk of tripping.

A further development of DPE Medical Ltd. (Israel) is described in US Patent No. 9,381,397.

### SUMMARY OF THE INVENTION

The present invention is a physiotherapeutic system combining stairs and an incline.

According to the teachings of an embodiment of the present invention there is provided, a system for deployment on an underlying surface, the system comprising: (a) a set of at least three horizontal tread surfaces including a first tread surface and a last tread surface; (b) a drive system mechanically linked to the set of tread surfaces and configured to displace at least two of the tread surfaces vertically so as to adjust a rise height between adjacent of the tread surfaces in such a manner as to form a set of stairs with uniform pitch for a range of different rise heights; and (c) a ramp having a first end hingedly connected to the last tread surface so as to define a walking surface from the first end to a second end of the ramp, the walking surface having a variable angle of inclination varying as a function of a vertical position of the last tread surface, wherein the drive system is configured to displace the tread surfaces to a fully-lowered state in which the set of tread surfaces are juxtaposed as a continuous flat surface, and further comprising a support configuration deployed to support the second end of the ramp over a range of motion such that, in the fully-lowered state, the second end of the ramp is supported above the underlying surface with the walking surface horizontal, the plurality of tread surfaces and the walking surface together forming a continuous horizontal walkway.

According to a further feature of an embodiment of the present invention, the drive system comprises a linear actuator deployed for raising the last tread surface vertically, the linear actuator being deployed outside an area of the walkway.

According to a further feature of an embodiment of the present invention, the continuous horizontal walkway is at a first height above the underlying surface, the system further comprising a connecting ramp having an upper edge hingedly connected to the second end of the ramp, the connecting ramp terminating at a lower edge adjacent to the underlying surface.

According to a further feature of an embodiment of the present invention, the drive system is configured to displace the tread surfaces so as to form the set of stairs with a plurality of rise heights substantially spanning a majority of a range from 0 cm to 18 cm.

According to a further feature of an embodiment of the present invention, the drive system is configured to displace the tread surfaces in a purely vertical motion, and wherein a plurality of the tread surfaces each has an associated vertical riser surface.

According to an additional feature of an embodiment of the present invention, there is also provided: (a) a set of sensors including: (i) a first sensor deployed to sense the presence of a patient on the first tread surface, (ii) a second sensor deployed to sense the presence of a subject on the last tread surface, and (iii) a third sensor deployed to sense the presence of a subject on the ramp in a region adjacent to the second end; and (b) a computer system comprising at least one processor and a non-volatile storage medium, the computer system being in communication with the set of sensors, the computer being configured to generate a data record of a patient activity, wherein the patient activity is determined at least in part from a sequence of outputs of the first, second and third sensors.

According to a further feature of an embodiment of the present invention, the set of sensors further comprises a rise height sensor deployed to generate an output indicative of a current rise height of the set of steps and a current inclination of the ramp, the computer being configured to perform at least two of the following: (a) on sensing of a patient at the first sensor followed by the second sensor at non-zero rise height, to identify a time from sensing of the first sensor to sensing of the second sensor as a time taken for ascending stairs at the currently sensed rise height; (b) on sensing of a patient at the third sensor followed by the second sensor at non-zero rise height, to identify a time from sensing of the third sensor to sensing of the second sensor as a time taken for ascending a slope at an inclination corresponding to the currently sensed rise height; and (c) on sensing of a zero rise height, identify a time from sensing of a patient at the first sensor to sensing of a patient at the third sensor, or the reverse, as a time taken to walk a predefined horizontal distance.

### BRIEF DESCRIPTION OF THE DRAWINGS

The invention is herein described, by way of example only, with reference to the accompanying drawings, wherein:
FIG. 1 is an isometric view of a therapeutic system, constructed and operative according to the teachings of an embodiment of the present invention, providing variable-rise steps and a variable-incline ramp, the system being shown in a fully-raised state;
FIG. 2 is an isometric view of the therapeutic system of FIG. 1, the system being shown in a fully-lowered state;
FIG. 3 is an isometric view of one of the steps from the system of FIG. 1 inverted to reveal an internal mechanism;
FIG. 4 is a view similar to FIG. 1 but taken from the ramp side of the system;
FIGS. 5A and 6A are views similar to FIG. 4 with selected elements removed to reveal internal components of the system;
FIGS. 5B and 6B are enlarged views of the regions of FIGS. 5A and 6A labeled V and VI, respectively;
FIG. 7 is a block diagram of the system of FIG. 1;
FIG. 8 is a schematic representation of various different modes of use of the system of FIG. 1; and
FIG. 9 is a schematic representation of a graphic user interface presenting data recorded by the system of FIG. 1 during a course of treatment for a give patient.

### DESCRIPTION OF THE PREFERRED EMBODIMENTS

The present invention is a physiotherapeutic system combining stairs and an incline, and corresponding methods for tracking and/or documenting therapy performed using the device.

The principles and operation of therapeutic systems according to the present invention may be better understood with reference to the drawings and the accompanying description.

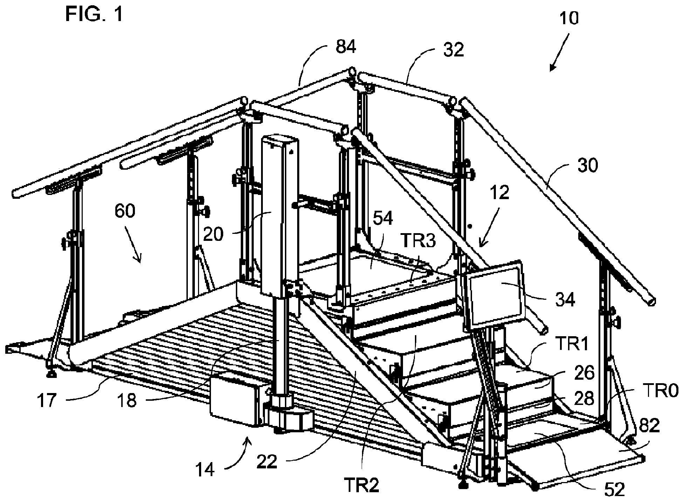

Referring now to the drawings, Figures 1-7 show various full or partial views of a physiotherapeutic stair training system, generally designated 10, constructed and operative according to a particularly preferred embodiment of the present invention.

The structural basis of system 10 according to this particularly preferred example employs a stair structure and lifting mechanism generally similar to that of US Patent No. 9,381,397 to DPE Medical Ltd. (Israel). Thus, the device has a set **12** of at least three, and in the case shown here four, horizontal tread surfaces, individually referred to as **TR0, TR1, TR2** and **TR3.** A drive system **14** is mechanically linked to the set **12** of tread surfaces and configured to displace at least two of the tread surfaces **TR1-TR3** vertically so as to adjust a rise height between adjacent of the tread surfaces in such a manner as to form a set of stairs **12** with uniform pitch for a range of different rise heights.

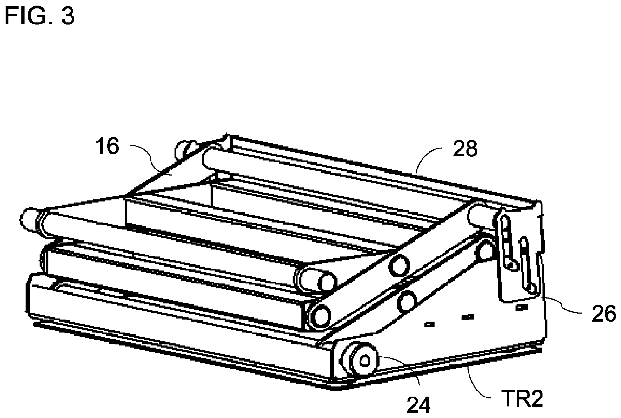

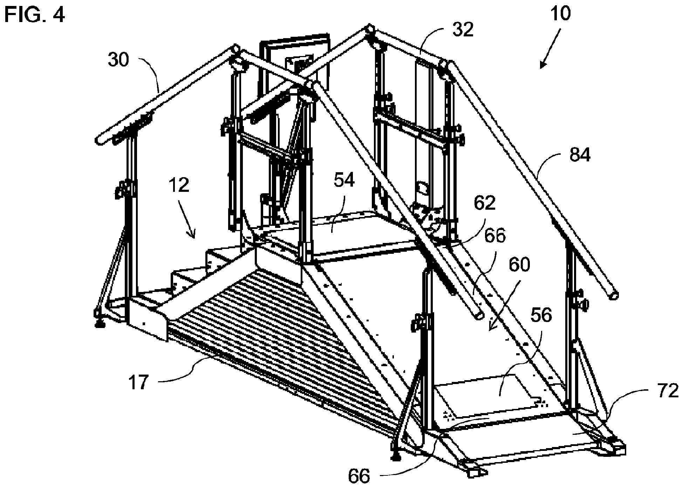

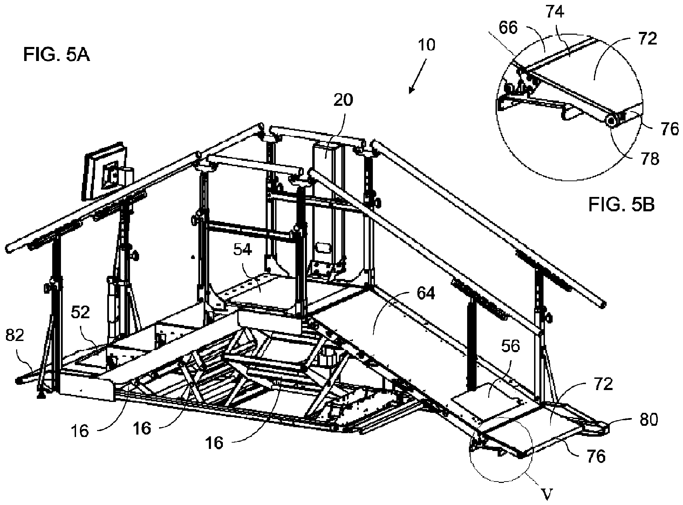

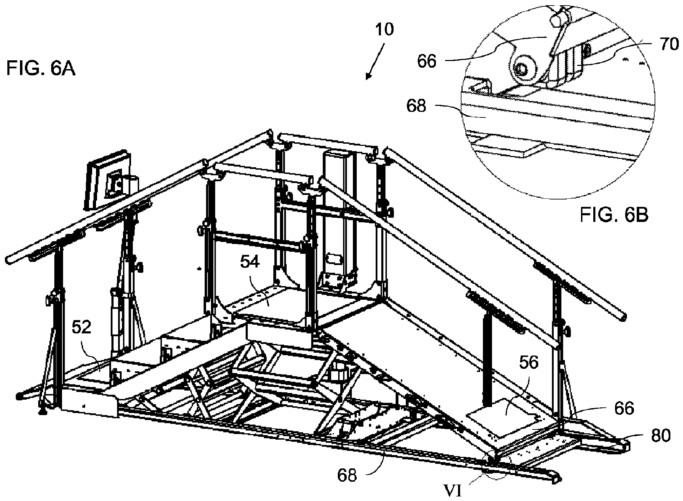

A preferred but non-limiting implementation of drive system **14** is best seen in FIG. 5A. Under each tread surface is a scissors mechanism **16,** which allows vertical movement of the tread surface while ensuring that it remains parallel to the floor. A linear drive, shown here in the form of a screw actuator **18,** is deployed in a column **20** beside top tread surface TR3 and deployed to move tread surface TR3 vertically. Other types of actuators, such as hydraulic or pneumatic actuators, may also be used. Two side rails **22** are pivotally linked to top tread surface **TR3** so as to be lifted at one end as the top tread surface rises. Each of the other tread surfaces has lateral projections **24** (FIG. 3) which engage rails **22** as a track, thereby lifting each tread surface in proportion to the elevation of the top surface and maintaining a set of stairs **12** of uniform pitch. In FIGS. 1 and 4, the scissors mechanisms are hidden by a folding protective skirt **17** to prevent objects or persons from being caught in the mechanisms, the protective skirt having been removed in FIGS. 5A and 6A to reveal the structural details.

It is important for stair therapy treatment that the steps are closed steps, i.e., with closed riser surfaces, so that the toes of a patient do not get caught beneath the step. To this end, each tread surface (other than TR0) is generally integrally formed with a riser surface **26** to form a step structure. Drive system **14** is preferably configured to displace the tread surfaces in a purely vertical motion, such as through the aforementioned scissors mechanisms **16.**

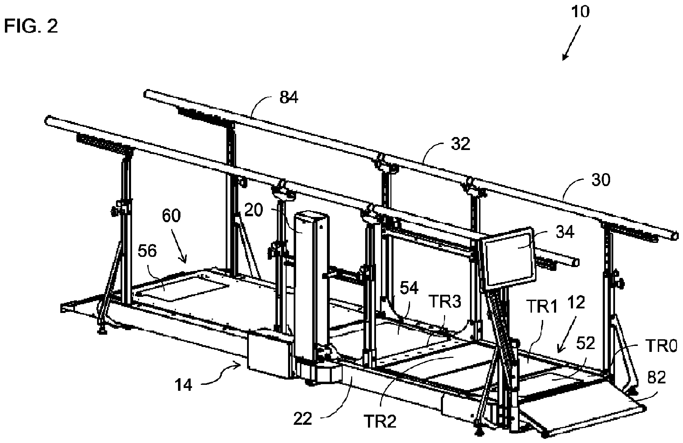

In the particularly preferred case illustrated in FIG. 3, each step also includes a telescopic extension portion **28** which provides an extension to riser surface **26** in the higher raised positions of the system, such as is seen in FIG. 1. The use of a telescopic extension portion allows the system to assume a fully lowered state (FIG. 2) in which a spacing from the floor is less than the maximum rise height of the set of stairs **12** in the fully raised state. By way of example, certain particularly preferred implementations of the present invention provide an adjustable step rise up to in excess of 16 centimeters, and preferably up to at least 18 centimeters, while the tread surfaces in the fully-lowered state are preferably less than 15 centimeters above the underlying surface, and most preferably no more than about 12 centimeters above the underlying surface.

The system preferably also includes at least one handrail extending alongside the set of stairs **12.** In the implementation illustrated here, an adjustable-height handrail **30** is provided on each side of the set of stairs **12,** and is complemented by handrail portions **32** extending along the top tread surface, which is here extended to form an upper platform to facilitate turning around between the ascent and descent when required. The handrail may additionally or alternatively be adjustable in horizontal position, to allow adjustment of the spacing between the right and left rails.

Drive system **14** is preferably configured to displace the tread surfaces so as to form the set of stairs **12** with a plurality of rise heights substantially spanning a majority of a range from 0 cm to 18 cm. "Substantially spanning" in this context refers to adjustability which provides either continuous adjustment or a plurality of discrete positions which are spaced apart by no more than 2 cm, and more typically in steps of 1 cm or less. In a particularly preferred implementation, the system provides adjustment to substantially span the entirety of a range of at least 0-18 cm, thereby facilitating practice of all common step sizes.

The system preferably assumes a fully flattened (zero step) state, as illustrated in FIG. 2. In this stage, the plurality of tread surfaces are juxtaposed as a continuous flat surface.

The present invention makes available an additional form of activity in the form of an adjustable ramp **60** (also referred to herein as a "slope" or "incline") for assessing and practicing the patient's ability to ascend and/or descend a slope. Ramp **60** has a first end **62** hingedly connected to the last tread surface **TR3** so as to define a walking surface **64** from first end **62** to a second end **66** of the ramp. Walking surface **64** has a variable angle of inclination varying as a function of a vertical position of the last tread surface **TR3.**

It is a particular feature of certain preferred embodiments of the present invention that there is provided a support configuration deployed to support second end **66** of ramp **60** over a range of motion such that, in the fully-lowered state of FIG. 2, second end **66** of ramp **60** is supported above the underlying surface with its walking surface **64** horizontal so that the plurality of tread surfaces **TR0, TR1, TR2, TR3** and walking surface **64** together form a continuous horizontal walkway. As described above, the various components such as scissor mechanisms **16** and riser surfaces **26** dictate a minimum height above the underlying surface at which the "zero" rise flat surface of the steps can be provided. If second end **66** of ramp **60** were to extend to the floor level, this would result in a small residual slope. The support configuration of this aspect of the present invention addresses this issue by ensuring that the orientation of walking surface **64** in the zero-rise state is parallel to the underlying surface, thus contributing to an extended horizontal walkway along the length of device **10,** preferably of at least 2.5 meters, and typically at least about 3 meters long.

The support configuration may take a wide range of forms, and may define various different loci of motion of second end **66** as the angle of the ramp varies. In a particularly simple and effective implementation as best seen in FIG. 6A and 6B, device 10 is formed with a pair of rails 68, which in the case illustrated here are elements of a device frame extending along the entire length of the device, along which second end 66 slides supported by sliding bearings 70. Sliding bearings may be preferred over rolling bearings due to the relatively large contact area and consequent spreading of load, although roller bearings may also be used.

In order to facilitate patients accessing ramp 60, a connecting ramp 72 is preferably provided with an upper edge **74** hingedly connected to second end **66** of ramp **60,** as best seen in FIG. 5B. Connecting ramp **72** terminates at a lower edge **76** adjacent to the underlying surface. "Adjacent" in this context indicates that lower edge **76** is sufficiently close to the underlying surface that a patient's foot cannot get caught beneath it. Preferably lower edge **76** is also sufficiently thin that it does not present a significant step to mount the leading edge. The leading edge is therefore preferably no more than about 3 centimeters thick, and more preferably no more than about 2 centimeters thick. In order to minimize friction as the lower edge moves along the underlying surface during vertical motion of the first end **62** of ramp **60** and corresponding horizontal motion of second end **66,** lower edge **76** of connecting ramp **72** is preferably also mounted on bearings, shown here as roller bearings **78,** which may roll directly on the underlying surface or, more preferably, on a thin inward-projecting lip **80** from rails **68.**

A second connecting ramp **82** it typically provided at the end of the device opposite to connecting ramp **72** to help patients mount the first step **TR0.** Step **TR0** and connecting ramp **82** are typically fixed, static components.

In order to minimize the height of the device above the underlying surface when fully lowered, the linear actuator **18** deployed for raising the device vertically is preferably deployed outside the area of the walkway, as illustrated, and most typically adjacent to a side of the upper platform, corresponding to **TR3.**

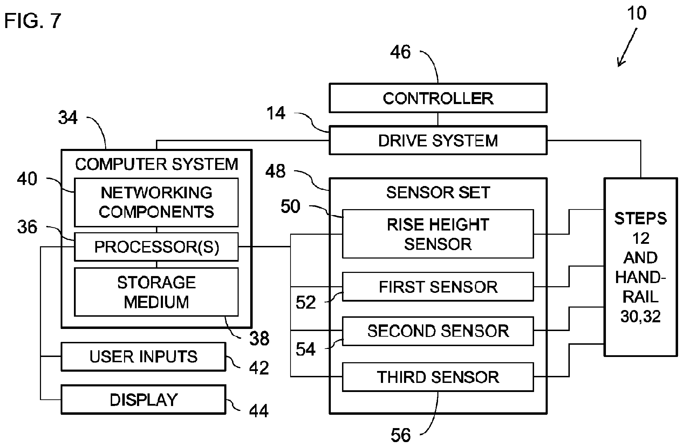

An elevation sensor or "rise height sensor" **50** (FIG. 7, typically implemented as a linear encoder directly measuring the platform height, or a linear or rotary encoder tracking motion of the actuator) gives an indication of the position of the platform, and hence also of the step rise, at any time.

As a result of the structure described above, adjustment of the height of the central platform simultaneously adjusts both the uniform step height and the angle of inclination of the slope, making available two distinct modes of therapeutic activity. The output of the elevation sensor also indicates the current angle of inclination of the slope.

In addition to the aforementioned components, as illustrated in the non-limiting example of FIG. 7, the present invention features a computer system **34** comprising at least one processor **36** and a non-volatile data storage medium **38,** typically together with networking components **40** for connection to a wired or wireless network. Computer system **34** may be any type of suitable computer, including but not limited to, a general purpose computer running suitable software under a suitable operating system, and a dedicated computer system configured by suitable hardware, software and/or firmware to perform the various functions required. In some implementations, computer system **34** may be implemented using a mobile electronics device such as a smartphone operating suitable software and in wireless communication with the various other components of the system.

Computer system **34** is preferably associated with, and possibly integrated with, one or more user input device **42** and a display **44.** In one preferred case, the entire computer system **34** is integrated in a "tablet" configuration with a touch-screen which serves as both the input device and the display, as illustrated schematically in FIG. 1.

Control of the up/down motion of drive system **14** may be achieved by pressing directly on up/down buttons on a controller **46** associated with drive system **14.** Additionally, or alternatively, control of drive system **14** may be achieved via the user interface of computer system **34.**

Computer system **34** is preferably associated with a sensor set **48** including at least one sensor deployed for measuring a parameter related to physiotherapy performed using device **10.** Preferred examples of the sensors, and corresponding modes of operation of the system, are described below.

According to certain particularly preferred implementations of the present invention, the device is provided with at least three sensors for sensing the presence of the patient at specific locations on the device. These sensors preferably include a first sensor **52** deployed for sensing the presence of the patient on the first (lowest) step **TR0;** a second sensor **54** deployed for sensing the presence of the patient on the central platform **TR3;** and a third sensor **56** deployed for sensing the presence of the patient adjacent to the bottom end of the ramp **60.** The sensors may advantageously be implemented as pressure sensors built into the surfaces on which the user steps, although any other suitable type of sensors may be used, including but not limited to: optical sensors, proximity sensors, RFID-based sensing arrangements, and image-processing-based detection.

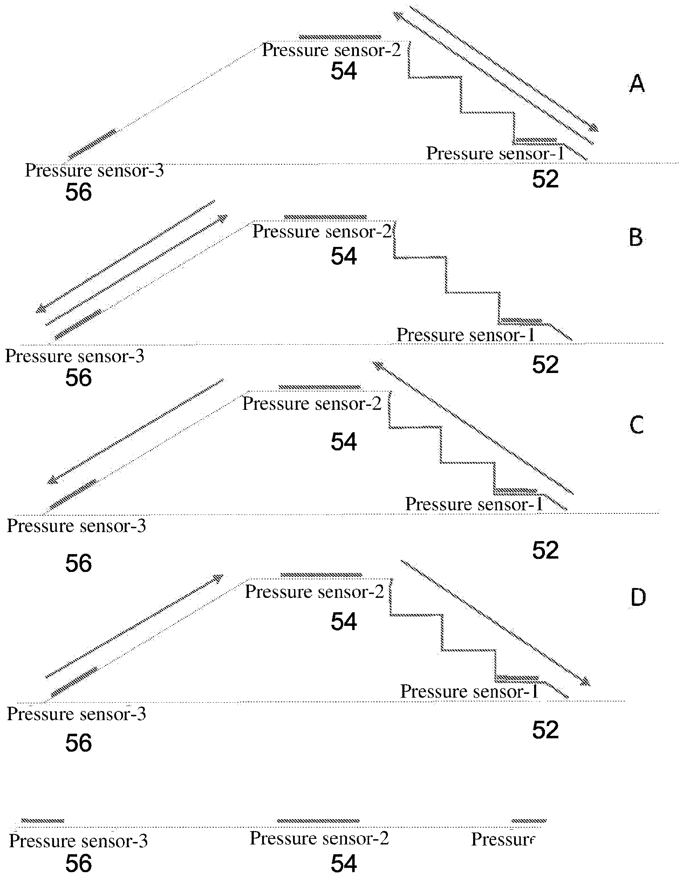

The deployment of the first, second and third sensors **52, 54** and **56** as described herein provides valuable functionality, as the system can automatically identify and record the type(s) of activity performed during a treatment session. This is illustrated in FIG. 8. Specifically, the order in which the sensors detect the presence of the patient indicates what combination of stair or slope climbing or descent has been performed, as follows:

| Case | Order | Activity |
|---|---|---|
| A | 1-2-1 | Ascend stairs; descend stairs |
| B | 3-2-3 | Ascend slope; descend slope |
| C | 1-2-3 | Ascend stairs; descend slope |
| D | 3-2-1 | Ascend slope; descend stairs |
| C or D | 1-2-3 or 3-2-1 | [Zero elevation] Walking |

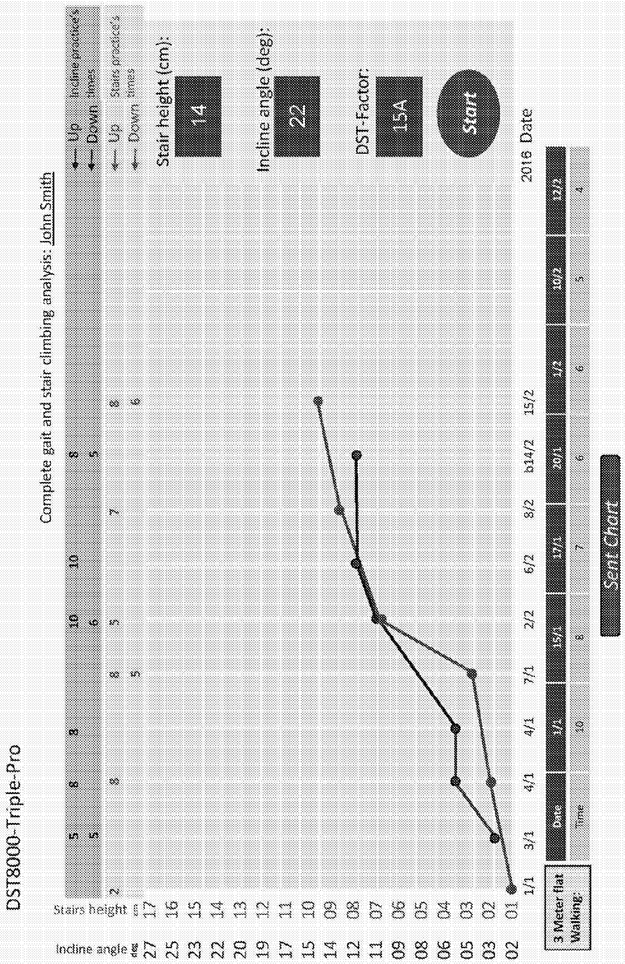

In addition to identifying the type of activity performed, the time from leaving one sensor until reaching the next gives a measure of the time taken on that occasion to complete the identified activity. This information is preferably stored together with the step rise or incline gradient/angle as part of the patient's personal record of treatment and performance (using data storage and recall functionality provided by computer system **34** directly and/or via remote storage accessed via networking components **40),** and can then be used to generate a graphic display and/or printed chart or graph of the patient's progress over a course of treatment, facilitating monitoring of the progress of treatment. By way of a non-limiting illustrative example, an on-screen display showing a patient's progress is illustrated in FIG. 9.

Parenthetically, it may be noted in FIG. 9 that the records of "UP" activities and "DOWN" activities in each therapy session do not necessarily balance. This occurs when a healthcare professional determines that the patient should focus on one or another activity, in which case the height of the device may be reduced or zeroed between ascending and descending, and the minor activity may not be recorded. Additionally, even where data exists for all activities of ascending/descending stairs/slope, it may be preferable to selectively display only part of the data in graphic form at any given time in order to avoid over-cluttering the display. Most preferably, a graphic user interface allows the healthcare professional to select the data to be displayed, for example, displaying plots of progress for ascending stairs and slope without descents, or displaying ascending and descending stairs only. Any and all other combinations may also be used.

Also included in the analytical data is a record of the level-surface walking time between sensors 1 and 3 (in either direction) which records the time taken for the patient to walk a predefined distance on a flat surface, in this case, about 3 meters. Walking ability is a further important indicator of the readiness of a patient to return to independent functioning outside a healthcare environment, and many treatment environments lack arrangements for accurately measuring and recording the level-surface walking ability of a patient. The combination step-and-incline device of the present invention thus provides significant added value by providing a tool for additionally monitoring and recording the level-surface walking ability of patients.

The device preferably includes handrails on both sides of the device, which are subdivided into rails **32** of the central platform, rails **30** for the stairs and rails **84** for ramp **60.** The handrails preferably also extend to or beyond connecting ramps **72** and **82.** The handrails for the stairs and ramp are preferably hingedly interconnected with supports for the central platform handrails, and are mounted via pin-in-slot engagements, or some other linear-bearing arrangement, to poles near the extremities of device **10,** to accommodate changes in the length of handrail between the supports as the elevation is adjusted. The handrails are preferably supported by adjustable supports to allow for adjustment for patients of different heights, such as for adults and children. A mechanism may also be provided (not shown) to allow adjustment of the horizontal position of the handrails, and thus adjust the span between the handrails.

The system preferably includes or interfaces with additional components including, but not limited to, control system components, user interface components, networking components and remote computer systems, and/or additional sensors, to provide additional functionality. By way of non-limiting examples, the various sensors for weight distribution, heart rate etc. described in the aforementioned US Patent No. 9,381,397 may all be implemented to advantage in the context of the present invention.

It will be appreciated that the above descriptions are intended only to serve as examples, and that many other embodiments are possible within the scope of the present invention as defined in the appended claims.

## Claims

1. A system (10) for deployment on an underlying surface, the system comprising:
(a) a set (12) of at least three horizontal tread surfaces (TR0, TR1, TR2, TR3) including a first tread surface (TR0) and a last tread surface (TR3); and
(b) a drive system (14) mechanically linked to said set (12) of tread surfaces and configured to displace at least two of said tread surfaces vertically so as to adjust a rise height between adjacent of said tread surfaces in such a manner as to form a set of stairs with uniform pitch for a range of different rise heights,
wherein said drive system (14) is configured to displace said tread surfaces to a fully-lowered state in which said set of tread surfaces are juxtaposed as a continuous flat surface,
**characterized in that** the system further comprises:
(c) a ramp (60) having a first end (62) hingedly connected to said last tread surface (TR3) so as to define a walking surface (64) from said first end (62) to a second end (66) of said ramp, said walking surface having a variable angle of inclination varying as a function of a vertical position of said last tread surface; and
(d) a support configuration (68, 70) deployed to support said second end (66) of said ramp over a range of motion such that, in said fully-lowered state, said second end of said ramp is supported above the underlying surface with said walking surface (64) horizontal, said plurality of tread surfaces (TRO, TR1, TR2, TR3) and said walking surface (64) together forming a continuous horizontal walkway.

2. The system (10) of claim 1, wherein said drive system (14) comprises a linear actuator (18) deployed for raising said last tread surface (TR3) vertically, said linear actuator being deployed outside an area of said walkway.

3. The system (10) of claim 1, wherein said continuous horizontal walkway is at a first height above the underlying surface, the system further comprising a connecting ramp (72) having an upper edge (74) hingedly connected to said second end (66) of said ramp, said connecting ramp (72) terminating at a lower edge (76) adjacent to the underlying surface.

4. The system (10) of claim 1, wherein said drive system (14) is configured to displace said tread surfaces (TRO, TR1, TR2, TR3) so as to form said set of stairs with a plurality of rise heights substantially spanning a majority of a range from 0 cm to 18 cm.

5. The system (10) of claim 1, wherein said drive system (14) is configured to displace said tread surfaces (TRO, TR1, TR2, TR3) in a purely vertical motion, and wherein a plurality of said tread surfaces each has an associated vertical riser surface (26).

6. The system (10) of claim 1, further comprising:
(a) a set (48) of sensors including:
(i) a first sensor (52) deployed to sense the presence of a patient on said first tread surface (TRO),
(ii) a second sensor (54) deployed to sense the presence of a subject on said last tread surface (TR3), and
(iii) a third sensor (56) deployed to sense the presence of a subject on said ramp (60) in a region adjacent to said second end (66); and
(b) a computer system (34) comprising at least one processor (36) and a non-volatile storage medium (38), said computer system being in communication with said set (48) of sensors, said computer being configured to generate a data record of a patient activity, wherein said patient activity is determined at least in part from a sequence of outputs of said first, second and third sensors (52, 54, 56).

7. The system (10) of claim 6, wherein said set of sensors further comprises a rise height sensor (50) deployed to generate an output indicative of a current rise height of said set (12) of steps and a current inclination of said ramp (60), said computer system (34) being configured to perform at least two of the following:
(a) on sensing of a patient at said first sensor (52) followed by said second sensor (54) at non-zero rise height, to identify a time from sensing of said first sensor to sensing of said second sensor as a time taken for ascending stairs at the currently sensed rise height;
(b) on sensing of a patient at said third sensor (56) followed by said second sensor (54) at non-zero rise height, to identify a time from sensing of said third sensor to sensing of said second sensor as a time taken for ascending a slope at an inclination corresponding to the currently sensed rise height; and
(c) on sensing of a zero rise height, identify a time from sensing of a patient at said first sensor (52) to sensing of a patient at said third sensor (56), or the reverse, as a time taken to walk a predefined horizontal distance.

8. The system (10) of claim 1, **characterized in that** the system further comprises:
(d) a set (48) of sensors including:
(i) a first sensor (52) deployed to sense the presence of a patient on said first tread surface (TRO),
(ii) a second sensor (54) deployed to sense the presence of a subject on said last tread surface (TR3), and
(iii) a third sensor (56) deployed to sense the presence of a subject on said ramp (60) in a region adjacent to said second end (66); and
(e) a computer system (34) comprising at least one processor (36) and a non-volatile storage medium (38), said computer system being in communication with said set of sensors, said computer being configured to generate a data record of a patient activity, wherein said patient activity is determined at least in part from a sequence of outputs of said first, second and third sensors (52, 54, 56).

9. The system (10) of claim 8, wherein said set (48) of sensors further comprises a rise height sensor (50) deployed to generate an output indicative of a current rise height of said set (12) of steps and a current inclination of said ramp (60), said computer system (34) being configured to perform at least two of the following:
(a) on sensing of a patient at said first sensor (52) followed by said second sensor (54) at non-zero rise height, to identify a time from sensing of said first sensor to sensing of said second sensor as a time taken for ascending stairs at the currently sensed rise height;
(b) on sensing of a patient at said third sensor (56) followed by said second sensor (54) at non-zero rise height, to identify a time from sensing of said third sensor to sensing of said second sensor as a time taken for ascending a slope at an inclination corresponding to the currently sensed rise height; and
(c) on sensing of a zero rise height, identify a time from sensing of a patient at said first sensor (52) to sensing of a patient at said third sensor (56), or the reverse, as a time taken to walk a predefined horizontal distance.

## Patentansprüche

1. System (10) zur Bereitstellung auf einer darunter liegenden Oberfläche, wobei das System umfasst:
(a) einen Satz (12) von mindestens drei horizontalen Trittflächen (TRO, TR1, TR2, TR3), einschließlich einer ersten Trittfläche (TR0) und einer letzten Trittfläche (TR3); und
(b) ein Antriebssystem (14), das mechanisch mit dem Satz (12) von Trittflächen verbunden und konfiguriert ist, mindestens zwei derTrittflächen vertikal zu verschieben, um eine Anstiegshöhe zwischen benachbarten Trittflächen derart anzupassen, dass ein Satz Stufen mit gleichmäßiger Steigung für verschiedene Höhen gebildet wird,
wobei das Antriebssystem (14) konfiguriert ist, die Trittflächen in einen vollständig abgesenkten Zustand zu versetzen, in dem der Satz von Trittfflächen als durchgehende flache Oberfläche nebeneinander angeordnet ist;
**dadurch gekennzeichnet, dass** das System ferner umfasst:
(c) eine Rampe (60) mit einem ersten Ende (62), das mit der letzten Trittfläche (TR3) gelenkig verbunden ist, um eine Lauffläche (64) von dem ersten Ende (62) zu einem zweiten Ende (66) der Rampe zu definieren; wobei die Lauftfläche einen variablen Neigungswinkel aufweist, der als Funktion einer vertikalen Position der letzten Trittfläche variiert; und
(d) eine Stützstruktur (68, 70), die eingesetzt wird, um das zweite Ende (66) der Rampe über einen Bewegungsbereich derart zu stützen, dass in dem vollständig abgesenkten Zustand das zweite Ende der Rampe über der darunter liegenden Oberfläche abgestützt wird, wobei die Lauffläche (64) horizontal ist und wobei die mehreren Trittflächen (TRO, TR1, TR2, TR3) und die Lauffläche (64) zusammen einen durchgehenden horizontalen Laufsteg bilden.

2. System (10) nach Anspruch 1, wobei das Antriebssystem (14) einen Linearantrieb (18) umfasst, der zum vertikalen Anheben der letzten Trittfläche (TR3) eingesetzt wird, wobei der Linearantrieb außerhalb eines Bereichs des Laufstegs eingesetzt ist.

3. System (10) nach Anspruch 1, wobei sich der durchgehende horizontale Laufsteg in einer ersten Höhe über der darunter liegenden Oberfläche befindet, wobei das System ferner eine Verbindungsrampe (72) mit einer Oberkante (74) umfasst, die mit dem zweiten Ende (66) der Rampe gelenkig verbunden ist, wobei die Verbindungsrampe (72) an einer Unterkante (76) neben der darunter liegenden Oberfläche endet.

4. System (10) nach Anspruch 1, wobei das Antriebssystem (14) konfiguriert ist, die Trittflächen (TRO, TR1, TR2, TR3) zu verschieben, um den Satz Treppen mit einer Vielzahl von Steighöhen zu bilden, die sich im Wesentlichen über eine Mehrheit in einem Bereich von 0 cm bis 18 cm erstrecken.

5. System (10) nach Anspruch 1, wobei das Antriebssystem (14) konfiguriert ist, die Trittflächen (TRO, TR1, TR2, TR3) in einer rein vertikalen Bewegung zu verschieben, und wobei mehreren der Trittflächen jeweils eine vertikale Steigfläche (26) zugeordnet ist.

6. System (10) nach Anspruch 1, ferner umfassend:
(a) einen Satz (48) von Sensoren, der Folgendes beinhaltet:
(i) einen ersten Sensor (52), der eingesetzt wird, um die Anwesenheit eines Patienten auf der ersten Trittfläche (TR0) zu erfassen,
(ii) einen zweiten Sensor (54), der eingesetzt wird, um die Anwesenheit einer Person auf der letzten Trittfläche (TR3) zu erfassen, und
(iii) einen dritten Sensor (56), der eingesetzt wird, um die Anwesenheit einer Person auf der Rampe (60) in einem Bereich neben dem zweiten Ende (66) zu erfassen; und
(b) ein Computersystem (34), das mindestens einen Prozessor (36) und ein nichtflüchtiges Speichermedium (38) umfasst, wobei das Computersystem mit dem Satz (48) von Sensoren in Verbindung steht, wobei der Computer konfiguriert ist, einen Datensatz einer Patientenaktivität zu erzeugen, wobei die Patientenaktivität zumindest teilweise aus einer Folge von Ausgaben des ersten, zweiten und dritten Sensors (52, 54, 56) bestimmt wird.

7. System (10) nach Anspruch 6, wobei der Satz von Sensoren ferner einen Anstiegshöhensensor (50) umfasst, der eingesetzt wird, um eine Ausgabe zu erzeugen, die eine aktuelle Anstiegshöhe des Satzes (12) von Stufen und eine aktuelle Neigung der Rampe (50) anzeigt, wobei das Computersystem (34) konfiguriert ist, mindestens zwei der Folgenden auszuführen:
(a) beim Erfassen eines Patienten an dem ersten Sensor (52), gefolgt von dem zweiten Sensor (54) bei einer Anstiegshöhe ungleich Null, Identifizieren einer Zeit vom Erfassen des ersten Sensors bis zum Erfassen des zweiten Sensors als eine Zeit, die für das Steigen der Stufen bei der aktuell erfassten Anstiegshöhe benötigt wird;
(b) beim Erfassen eines Patienten an dem dritten Sensor (56), gefolgt von dem zweiten Sensor (54) bei einer Anstiegshöhe ungleich Null, Identifizieren einer Zeit vom Erfassen des dritten Sensors bis zum Erfassen des zweiten Sensors als eine Zeit, die für das Steigen der Stufen bei der aktuell erfassten Anstiegshöhe benötigt wird;
(c) beim Erfassen einer Anstiegshöhe gleich Null, Identifizieren einer Zeit vom Erfassen eines Patienten an dem ersten Sensor (52) bis zum Erfassen eines Patienten an dem dritten Sensor (56) oder umgekehrt als eine Zeit, die benötigt wird, um eine vordefinierte horizontale Strecke zu gehen.

8. System (10) nach Anspruch 1,
**dadurch gekennzeichnet, dass** das System ferner umfasst:
(d) einen Satz (48) von Sensoren, der Folgendes beinhaltet:
(i) einen ersten Sensor (52), der eingesetzt wird, um die Anwesenheit eines Patienten auf der ersten Trittfläche (TR0) zu erfassen,
(ii) einen zweiten Sensor (54), der eingesetzt wird, um die Anwesenheit einer Person auf der letzten Trittfläche (TR3) zu erfassen, und
(iii) einen dritten Sensor (56), der eingesetzt wird, um die Anwesenheit einer Person auf der Rampe (60) in einem Bereich neben dem zweiten Ende (66) zu erfassen; und
(e) ein Computersystem (34), das mindestens einen Prozessor (36) und ein nichtflüchtiges Speichermedium (38) umfasst, wobei das Computersystem mit dem Satz von Sensoren in Verbindung steht, wobei der Computer konfiguriert ist, um einen Datensatz einer Patientenaktivität zu erzeugen wobei die Patientenaktivität zumindest teilweise aus einer Folge von Ausgaben des ersten, zweiten und dritten Sensors (52, 54, 56) bestimmt wird.

9. System (10) nach Anspruch 8, wobei der Satz (48) von Sensoren ferner einen Anstiegshöhensensor (50) umfasst, der eingesetzt wird, um eine Ausgabe zu erzeugen, die eine aktuelle Anstiegshöhe des Satzes (12) von Stufen und eine aktuelle Neigung der Rampe (50) anzeigt, wobei das Computersystem (34) konfiguriert ist, mindestens zwei der Folgenden auszuführen:
(a) beim Erfassen eines Patienten an dem ersten Sensor (52), gefolgt von dem zweiten Sensor (54) bei einer Anstiegshöhe ungleich Null, Identifizieren einer Zeit vom Erfassen des ersten Sensors bis zum Erfassen des zweiten Sensors als eine Zeit, die für das Steigen der Stufen bei der aktuell erfassten Anstiegshöhe benötigt wird;
(b) beim Erfassen eines Patienten an dem dritten Sensor (56), gefolgt von dem zweiten Sensor (54) bei einer Anstiegshöhe ungleich Null, Identifizieren einer Zeit vom Erfassen des dritten Sensors bis zum Erfassen des zweiten Sensors als eine Zeit, die für das Steigen der Stufen bei der aktuell erfassten Anstiegshöhe benötigt wird;
(c) beim Erfassen einer Anstiegshöhe gleich Null, Identifizieren einer Zeit vom Erfassen eines Patienten an dem ersten Sensor (52) bis zum Erfassen eines Patienten an dem dritten Sensor (56) oder umgekehrt als eine Zeit, die benötigt wird, um eine vordefinierte horizontale Strecke zu gehen.

## Revendications

1. Système (10) destiné à être déployé sur une surface sous-jacente, le système comprenant :
(a) un ensemble (12) d'au moins trois surfaces de plan de marche horizontales (TRO, TR1, TR2, TR3) comprenant une première surface de plan de marche (TR0) et une dernière surface de plan de marche (TR3) ; et
(b) un système d'entraînement (14) lié mécaniquement audit ensemble (12) de surfaces de plan de marche et conçu pour déplacer au moins deux desdites surfaces de plan de marche verticalement de manière à régler une hauteur d'élévation entre les surfaces adjacentes desdites surfaces de plan de marche de manière à former un ensemble d'escaliers à pas uniforme pour une plage de hauteurs d'élévation différentes,
ledit système d'entraînement (14) étant conçu pour déplacer lesdites surfaces de plan de marche vers un état complètement abaissé dans lequel ledit ensemble de surfaces de plan de marche sont juxtaposées sous la forme d'une surface plate continue,
**caractérisé en ce que** le système comprend en outre :
(c) une rampe (60) ayant une première extrémité (62) reliée avec articulation à ladite dernière surface de plan de marche (TR3) de manière à définir une surface de marche (64) de ladite première extrémité (62) à une seconde extrémité (66) de ladite rampe, ladite surface de marche ayant un angle d'inclinaison variable variant en fonction d'une position verticale de ladite dernière surface de plan de marche ; et
(d) une configuration de support (68, 70) déployée pour supporter ladite seconde extrémité (66) de ladite rampe sur une plage de mouvement de telle sorte que, dans ledit état complètement abaissé, ladite seconde extrémité de ladite rampe est supportée au-dessus de la surface sous-jacente avec ladite surface de marche (64) horizontale, ladite pluralité de surfaces de plan de marche (TRO, TR1, TR2, TR3) et ladite surface de marche (64) formant ensemble un passage horizontal continu.

2. Système (10) selon la revendication 1, dans lequel ledit système d'entraînement (14) comprend un actionneur linéaire (18) déployé pour élever ladite dernière surface de plan de marche (TR3) verticalement, ledit actionneur linéaire étant déployé à l'extérieur d'une zone dudit passage.

3. Système (10) selon la revendication 1, dans lequel ledit passage horizontal continu est à une première hauteur au-dessus de la surface sous-jacente, le système comprenant en outre une rampe de liaison (72) ayant un bord supérieur (74) relié avec articulation à ladite seconde extrémité (66) de ladite rampe, ladite rampe de liaison (72) se terminant à un bord inférieur (76) adjacent à la surface sous-jacente.

4. Système (10) selon la revendication 1, dans lequel ledit système d'entraînement (14) est conçu pour déplacer lesdites surfaces de plan de marche (TRO, TR1, TR2, TR3) de manière à former ledit ensemble d'escaliers avec une pluralité de hauteurs d'élévation couvrant sensiblement une majorité d'une plage de 0 à 18 cm.

5. Système (10) selon la revendication 1, dans lequel ledit système d'entraînement (14) est conçu pour déplacer lesdites surfaces de plan de marche (TRO, TR1, TR2, TR3) dans un mouvement purement vertical, et dans lequel une pluralité desdites surfaces de plan de marche comportent chacune une surface d'élévation verticale (26) associée.

6. Système (10) selon la revendication 1, comprenant en outre :
(a) un ensemble (48) de capteurs comprenant :
(i) un premier capteur (52) déployé pour détecter la présence d'un patient sur ladite première surface de plan de marche (TRO),
(ii) un deuxième capteur (54) déployé pour détecter la présence d'un sujet sur ladite dernière surface de plan de marche (TR3), et
(iii) un troisième capteur (56) déployé pour détecter la présence d'un sujet sur ladite rampe (60) dans une région adjacente à ladite seconde extrémité (66) ; et
(b) un système informatique (34) comprenant au moins un processeur (36) et un support de stockage non volatil (38), ledit système informatique étant en communication avec ledit ensemble (48) de capteurs, ledit ordinateur étant conçu pour générer un enregistrement de données d'une activité du patient, dans lequel ladite activité du patient est déterminée au moins en partie à partir d'une séquence de sorties desdits premier, deuxième et troisième capteurs (52, 54, 56).

7. Système (10) selon la revendication 6, dans lequel ledit ensemble de capteurs comprend en outre un capteur de hauteur d'élévation (50) déployé pour générer une sortie indiquant une hauteur d'élévation actuelle dudit ensemble (12) de marches et d'une inclinaison actuelle de ladite rampe (60), ledit système informatique (34) étant conçu pour effectuer au moins deux des opérations suivantes :
(a) lors de la détection d'un patient au niveau dudit premier capteur (52) suivi dudit deuxième capteur (54) à une hauteur d'élévation non nulle, l'identification d'un temps entre la détection dudit premier capteur et la détection dudit deuxième capteur comme un temps mis pour monter des escaliers à la hauteur d'élévation actuellement détectée ;
(b) lors de la détection d'un patient au niveau dudit troisième capteur (56) suivi dudit deuxième capteur (54) à une hauteur d'élévation non nulle, l'identification d'un temps entre la détection dudit troisième capteur et la détection dudit deuxième capteur comme un temps mis pour monter une pente à une inclinaison correspondant à la hauteur d'élévation actuellement détectée ; et
(c) lors de la détection d'une hauteur d'élévation nulle, l'identification d'un temps entre la détection d'un patient au niveau dudit premier capteur (52) et la détection d'un patient au niveau dudit troisième capteur (56), ou l'inverse, comme un temps mis pour parcourir une distance horizontale prédéfinie.

8. Système (10) selon la revendication 1,
**caractérisé en ce que** le système comprend en outre :
(d) un ensemble (48) de capteurs comprenant :
(i) un premier capteur (52) déployé pour détecter la présence d'un patient sur ladite première surface de plan de marche (TRO),
(ii) un deuxième capteur (54) déployé pour détecter la présence d'un sujet sur ladite dernière surface de plan de marche (TR3), et
(iii) un troisième capteur (56) déployé pour détecter la présence d'un sujet sur ladite rampe (60) dans une région adjacente à ladite seconde extrémité (66) ; et
(e) un système informatique (34) comprenant au moins un processeur (36) et un support de stockage non volatil (38), ledit système informatique étant en communication avec ledit ensemble de capteurs, ledit ordinateur étant conçu pour générer un enregistrement de données d'une activité du patient, dans lequel ladite activité du patient est déterminée au moins en partie à partir d'une séquence de sorties desdits premier, deuxième et troisième capteurs (52, 54, 56).

9. Système (10) selon la revendication 8, dans lequel ledit ensemble (48) de capteurs comprennent en outre un capteur de hauteur d'élévation (50) déployé pour générer une sortie indiquant une hauteur d'élévation actuelle dudit ensemble (12) de marches et 'une inclinaison actuelle de ladite rampe (60), ledit système informatique (34) étant conçu pour effectuer au moins deux des opérations suivantes :
(a) lors de la détection d'un patient au niveau dudit premier capteur (52) suivi dudit deuxième capteur (54) à une hauteur d'élévation non nulle, l'identification d'un temps entre la détection dudit premier capteur et la détection dudit deuxième capteur comme un temps mis pour monter des escaliers à la hauteur d'élévation actuellement détectée ;
(b) lors de la détection d'un patient au niveau dudit troisième capteur (56) suivi dudit deuxième capteur (54) à une hauteur d'élévation non nulle, l'identification d'un temps entre la détection dudit troisième capteur et la détection dudit deuxième capteur comme un temps mis pour monter une pente à une inclinaison correspondant à la hauteur d'élévation actuellement détectée ; et
(c) lors de la détection d'une hauteur d'élévation nulle, l'identification d'un temps entre la détection d'un patient au niveau dudit premier capteur (52) et la détection d'un patient au niveau dudit troisième capteur (56), ou l'inverse, comme un temps mis pour parcourir une distance horizontale prédéfinie.
